# EUROPÄISCHE PATENTSCHRIFT

(11) **EP 1 881 957 B1**
(45) Veröffentlichungstag und Bekanntmachung des Hinweises auf die Patenterteilung: **17.08.2011**
(21) Anmeldenummer: 06743390.4
(22) Anmeldetag: 02.05.2006
(51) Int. Cl.: C07C 227/26, C07C 229/16

(54) **VERFAHREN ZUR HERSTELLUNG VON NEBENPRODUKTARMEN METHYLGLYCIN-N,N-DIESSIGSÄURE-TRIALKALIMETALLSALZEN**
METHOD FOR PRODUCING METHYLGLYCINE-N,N-DIETHANOIC ACID-TRIALKALI METAL SALTS WITH A LOW BY-PRODUCT CONTENT
PROCEDE DE PRODUCTION DE SELS METALLIQUES TRIALCALINS D'ACIDE METHYLGLYCINE-N,N-DIACETIQUE PAUVRES EN SOUS-PRODUITS

(30) Priorität: 06.05.2005 DE 102005021055
(43) Veröffentlichungstag der Anmeldung: 30.01.2008
(73) Patentinhaber: BASF SE, 67056 Ludwigshafen (DE)
(72) Erfinder: OFTRING, Alfred, 67098 Bad Dürkheim (DE); BRAUN, Gerold, 67071 Ludwigshafen (DE); WIRSING, Friedrich, 67657 Kaiserslautern (DE); STAMM, Armin, 55268 Nieder-Olm (DE); BALDENIUS, Kai-Uwe, 69115 Heidelberg (DE)
(86) Internationale Anmeldenummer: PCT/EP2006/061968
(87) Internationale Veröffentlichungsnummer: WO 2006/120129

(56) Entgegenhaltungen:
- WO-A-94/29421
- US-A- 5 849 950

## Beschreibung

Die Erfindung betrifft ein Verfahren zur Herstellung von nebenproduktarmen, farbhellen Methylglycin-N,N-diessigsäure-Trialkalimetallsalzen durch alkalische Hydrolyse von Methylglycinnitril-N,N-diacetontril.

Die häufig als Komplexbildner beispielsweise in Reinigungsmitteln eingesetzten Aminopolyphosphonate, Polycarboxylate oder Aminopolycarboxylate, wie Ethylendiamintetraessigsäure (EDTA), sind nur in geringem Maße biologisch abbaubar. Eine preiswerte Alternative stellen die Glycin-N,N-diessigsäurederivate, wie die Methylglycin-N,N-diessigsäure (MGDA) dar, welche ungiftig und gut biologisch abbaubar ist. Die Verwendung von MGDA und von verwandten Glycin-N,N-diessigsäurederivaten in Reinigungsmitteln sowie deren Synthesen sind in WO-A 94/29421 und US 5,849,950 beschrieben. Für eine kostengünstige Produktion der Glycin-N,N-diessigsäurederivate werden hohe Anforderungen an die Ausbeute der einzelnen Syntheseschritte und Reinheit der isolierten Zwischenprodukte gestellt.

MGDA wird durch Umsetzung von Iminodiacetontril mit Acetaldehyd und Blausäure oder von alpha-Alaninnitril mit Formaldehyd und Blausäure und alkalische Hydrolyse des als Zwischenprodukt erhaltenen Methylglycinnitrildiacetontril (MGDN) mit Natronlauge hergestellt, wobei das Trinatriumsalz des MGDA erhalten wird. Um hohe MGDA-Ausbeuten und -Reinheiten zu erzielen, wird MGDN als Zwischenprodukt isoliert und als Reinstoff in dem sich anschließenden Hydrolyseschritt eingesetzt.

Problematisch bei der Hydrolyse von Alkylglycinnitril-N,N-diacetonitrilen ist ihre thermische Labilität, insbesondere im alkalischen Milieu. Durch die sterisch anspruchsvolle Alkylsubstitution werden Rückspaltungsreaktionen begünstigt. Im Falle von MGDN sind primäre Spaltprodukte insbesondere Cyanid, Acetaldehyd, Iminodiacetonitril (IDN) und Formaldehyd. Unter alkalischen Bedingungen können durch Hydrolyse- oder andere Nebenreaktionen darüber hinaus vor allem noch folgende Nebenprodukte (als Natrium- oder Kaliumsalze) entstehen: Iminodiacetat (IDA), Nitrilotriacetat (NTA), Carbonat, Acetat, Formiat, Glykolat, Lactat, Glycinat oder Alaninat. Cyanid ist akut toxisch. NTA ist als nierentoxisch beschrieben worden. Cyanid und Acetaldehyd neigen zur Polymerisation und können farbige Nebenprodukte bilden. Acetaldehyd kann darüber hinaus als flüchtige Komponente die Destillate des

Koppelproduktes der alkalischen Hydrolyse, Ammoniak, kontaminieren.

US 5,849,950 offenbart die Herstellung von Methylglycindiessigsäure durch Umsetzung von alpha-Alaninnitril mit Formaldehyd und Blausäure und alkalische Hydrolyse des als Zwischenprodukt erhaltenen Methylglycinnitrildiacetontril (MGDN) mit Natronlauge. Zur Hydrolyse wird kristallines MGDN bei 40°C in 20 gew.-%ige wässrige Natronlauge eingetragen, 3 h lang bei 40°C gerührt und anschließend noch 5 h lang bei 95°C weitergerührt. Bei der Hydrolyse werden in nicht unerheblichem Maße Nebenprodukte gebildet, beispielsweise NTA.

Aufgabe der Erfindung ist es, ein Verfahren zur Herstellung von nebenproduktarmem, farbhellem Methylglycin-N,N-diacetat bereitzustellen.

Gelöst wird die Aufgabe durch ein Verfahren zur Herstellung von nebenproduktarmem, farbhellem Methylglycin-N,N-diessigsäure-Trialkalimetallsalz durch alkalische Hydrolyse von Methylglycinnitrildiacetonitril mit den Schritten in der Reihenfolge (a) bis (f):
(a) Mischen von Methylglycinnitrildiacetontril (MGDN) mit wässriger Alkalilauge bei einer Temperatur ≤ 30°C;
(b) Reagieren lassen der wässrigen alkalischen MGDN-Suspension bei einer Temperatur im Bereich von 10 bis 30°C während eines Zeitraums von 0,1 bis 10 h, wobei eine Lösung gebildet wird;
(c) Reagieren lassen der Lösung aus Schritt (b) bei einer Temperatur im Bereich von 30 bis 40°C während eines Zeitraums von 0,1 bis 10 h;
(d) optional Reagieren lassen der Lösung aus Schritt (c) bei einer Temperatur im Bereich von 50 bis 80°C während eines Zeitraums von 0,5 bis 2 h;
(e) optional Reagieren lassen der Lösung aus Schritt (c) bzw. (d) bei einer Temperatur im Bereich von 110 bis 200°C während eines Zeitraums von 5 bis 60 min;
(f) Hydrolyse und Abtrennung von Ammoniak von der in Schritt (c), (d) bzw. (e) erhaltenen Lösung durch Strippen bei einer Temperatur von 90 bis 105°C.

In einem Schritt (a) wird Methylglycinnitrildiacetontril (MGDN) mit wässriger Alkalilauge bei einer Mischungstemperatur von ≤ 30°C gemischt. Im Allgemeinen beträgt die Mischungstemperatur 10 bis 30°C, bevorzugt 20 bis 25°C. MGDN kann als Feststoff, vorzugsweise als Pulver, als feuchte Maische oder als wässrige Suspension eingesetzt werden. Die wässrige Alkalilauge kann in einem geeigneten Mischbehälter (z. B. einem Rührreaktor) vorgelegt werden und MGDN als Feststoff oder wässrige Suspension zudosiert werden. Alkalilauge und MGDN können auch parallel in einen Mischbehälter oder einen Rohrreaktor dosiert werden. Als wässrige Alkalilauge kommen wässrige NaOH (Natronlauge) oder wässrige KOH (Kalilauge) mit einem Alkalimetallhydroxid-Gehalt von im Allgemeinen 5 bis 50 Gew.-%, vorzugsweise 20 bis 50 Gew.-% in Betracht. Bevorzugt ist Natronlauge. Das Molverhältnis MGDN zu Alkalilauge beträgt im Allgemeinen 1 : 3,0 - 3,5, bevorzugt 1 : 3,05 - 3,1.

Anschließend lässt man die wässrige alkalische MGDN-Lösung in Schritten (b) und (c) reagieren, wobei zwei verschiedene Temperaturstufen durchlaufen werden. Zunächst lässt man die wässrige alkalische MGDN-Lösung in einer ersten Stufe (b) bei einer Temperatur im Bereich von 20 bis 30°C, bevorzugt 25 bis 30°C, während eines Zeitraums von 0,1 bis 10 h, bevorzugt 1 bis 5 h, besonders bevorzugt 2 bis 3 h, und anschließend in einer zweiten Stufe (c) bei einer Temperatur im Bereich von 30 bis 40°C, bevorzugt 35 bis 40°C, während eines Zeitraums von 0,1 bis 10 h, bevorzugt 1 bis 5 h, besonders bevorzugt 3 bis 4 h reagieren.

In einem optionalen Schritt (d) wird die in Schritt (c) erhaltene Lösung noch bei einer Temperatur im Bereich von 50 bis 80°C, bevorzugt 70 bis 80°C während eines Zeitraums von 0,5 bis 2 h, bevorzugt 1 bis 2 h, reagieren gelassen.

An den Schritt (c) bzw. (d) kann sich eine so genannte Druckverseifung als Schritt (e) anschließen. Hierbei wird die erhaltene Lösung bei einer Temperatur im Bereich von 110 bis 200°C, bevorzugt von 140 bis 180°C, während eines Zeitraums von 5 bis 60 min verseift. Hierbei steht die Lösung unter einem der Temperatur entsprechenden (nautogenen") Druck.

Anschließend wird in einem Schritt (f) von der erhaltenen Lösung Ammoniak durch Strippen bei einer Temperatur von 90 bis 105°C, vorzugsweise von 95 bis 105°C abgetrennt. Dabei findet auch eine Rest-Hydrolyse der in Lösung enthaltenen hydrolysierbaren Komponenten unter Bildung von Ammoniak statt. Beispielsweise wird durch Druckemiedrigung auf 700 bis 960 mbar die Lösung weitgehend von Ammoniak frei gestrippt. Vorzugsweise wird zusätzlich noch Luft als Strippgas eingesetzt.

Die in Schritt (f) erhaltene Lösung kann anschließend zur weitgehenden bis vollständigen Entfärbung noch einem Bleichschritt unter Verwendung von Wasserstoffperoxid und/oder Aktivkohle als "Bleichmittel" unterzogen werden.

Die erfindungsgemäße MGDN-Hydrolyse kann diskontinuierlich, halbkontinuierlich oder kontinuierlich durchgeführt werden. Diskontinuierlich oder halbkontinuierlich wird die Hydrolyse beispielsweise in einem Rührreaktor durchgeführt, kontinuierlich wird sie beispielsweise in einer Kaskade aus Rührreaktoren und/oder in Rohrreakoren und/oder in Schlaufenreaktoren durchgeführt.

Das erfindungsgemäß erhältliche technische MGDA-Trinatriumsalz weist einen Nebenproduktgehalt von im Allgemeinen < 5 Gew.-%, bezogen auf den Feststoff, auf. Der Gehalt an NTA-Trinatriumsalz beträgt im Allgemeinen < 0,3 Gew.-%.

Ein MGDN enthaltendes wässriges Rohproduktgemisch kann erhalten werden durch
1. Umsetzung von Iminodiacetonitril (IDN) mit HCN und Acetaldehyd in wässriger Lösung. Iminodiacetonitril kann in einer vorgelagerten Stufe aus Urotropin und Blausäure oder aus Formaldehydcyanhydrin und Ammoniak als wässrige Emulsion erhalten werden;
2. Umsetzung von Alaninnitril mit HCN und Formaldehyd in wässriger Lösung. Alaninnitril kann in einer vorgelagerten Stufe aus Acetaldehyd, HCN und Ammoniak oder Acetaldehydcyanhydrin und Ammoniak erhalten werden. Vorzugsweise wird ein MGDN enthaltendes wässriges Rohproduktgemisch wie folgt erhalten:
1a. Iminodiaoetonitril (IDN) wird durch Umsetzung von Urotropin, das in situ aus Ammoniak und Formaldehyd erzeugt werden kann, mit Blausäure bei einem pH-Wert von 5,5 bis 6,3 und einer Temperatur im Bereich von 20 bis 90°C erhalten. Das Molverhältnis Ammoniak : Formaldehyd : Blausäure beträgt im Allgemeinen 1 : 1,5 : 1,5-1,9, die IDN-Konzentration in der erhaltenen wässrigen Emulsion beträgt im Allgemeinen 15-40 Gew.-%. Anschließend wird der pH-Wert der wässrigen IDN-Emulsion mit Mineralsäure auf 2 - 1,0 eingestellt. Die angesäuerte IDN-Emulsion wird dann mit Acetaldehyd und Blausäure zum MGDN umgesetzt. Das Molverhältnis IDN : Acetaldehyd : HCN beträgt im Allgemeinen 1 : 1-1,2 : 1 - 1,2, die Temperatur bei der Umsetzung beträgt im Allgemeinen 40 - 90°C. Die MGDN-Konzentration der erhaltenen wässrigen Emulsion beträgt im Allgemeinen 20 - 50 Gew.-%.
   IDN kann auch durch Umsetzung von Formaldehydcyanhydrin mit Ammoniak hergestellt werden. Alternativ kann von kristallinem IDN als Edukt ausgegangen werden, welches in Wasser suspendiert wird.
2a. Alpha-Alaninnitril (AN) wird durch Umsetzung von überschüssigem Ammoniak mit Acetaldehyd und HCN oder durch Umsetzung von Acetaldehydcyanhydrin mit überschüssigem Ammoniak herstellt, wobei Ammoniak als wässrige Lösung, gasförmig oder in flüssiger Form eingesetzt werden kann. Die Umsetzung kann unter Druck durchgeführt werden. Der überschüssige Ammoniak wird bevorzugt im Vakuum abdestilliert. Das rohe AN wird mit Formaldehyd und Blausäure zu MGDN umsetzt. Dazu wird der pH-Wert der wässrigen AN-Lösung mit Mineralsäure auf 2 - 1,0 eingestellt. Das Molverhältnis AN : Formaldehyd : HCN beträgt im Allgemeinen 1 : 1,0 - 1,2 : 1,0 - 1,2, die Temperatur bei der Umsetzung beträgt im Allgemeinen 40 - 90°C.

Die MGDN-Konzentration der erhaltenen wässrigen Emulsion beträgt im Allgemeinen 20 - 50 Gew.-%. Aus dieser kann MGDN durch Kristallisation abgetrennt werden. Vorzugsweise wird die wässrige Emulsion hierzu vor Durchführung der Kristallisation mit Wasser auf einen MGDN-Gehalt von 15 - 40 Gew.-% verdünnt.

Es kann auch das erhaltene wässrige, MGDN enthaltende Rohgemisch erfindungsgemäß alkalisch hydrolysiert werden. Auch dabei werden durch die alkalische Hydrolyse des MGDN weniger Nebenprodukte gebildet. Diese Variante ist wegen des erheblichen Nebenproduktgehalts des MGDN-Rohgemischs jedoch weniger bevorzugt. Vorzugsweise wird daher MGDN zunächst durch Kristallisation und Fest/flüssig-Trennung von dem Rohgemisch abgetrennt.

In einer bevorzugten Ausführungsform der Kristallisation wird das MGDN enthaltende Rohproduktgemisch, das im Allgemeinen als Emulsion von MGDN in einer gesättigten wässrigen MGDN-Lösung vorliegt, nur sehr langsam, also mit einer im zeitlichen Mittel geringen Abkühlrate (ausgedrückt in K/h) unter den Erstarrungspunkt abgekühlt. Erst wenn praktisch die Gesamtmenge des emulgierten MGDN erstarrt ist, wird vorzugsweise mit einer größeren Abkühlrate abgekühlt. Das dann aus der wässrigen Lösung auskristallisierende, gelöste MGDN trifft auf bereits erstarrtes, kristallines MGDN, wodurch die Neubildung von Kristallkeimen reduziert oder weitgehend ganz unterdrückt wird. Es wird daher deutlich weniger oder im Wesentlichen überhaupt kein Feinanteil gebildet. Während des Kristallisationsvorgangs wird Wasser verdampft, wobei dieser Verdampfungsvorgang mit einer Abkühlung und/oder Aufkonzentrierung des Gemischs einhergehen kann. Durch die Verdampfung entsteht, angrenzend an die Grenzfläche Flüssigkeit/Gasraum des wässrigen Gemischs, eine Zone der Übersättigung. In dieser Zone der Übersättigung können Kristalle gebildet werden, welche anschließend in das Innere der Flüssigkeit transportiert werden und dort weiter wachsen. Da wegen der langsamen Verdampfung nur in der sehr schmalen Zone der Übersättigung im Wesentlichen unterhalb der Flüssigkeitsoberfläche neue Kristalle gebildet werden und nur diese im Innern der Flüssigkeit weiter wachsen, werden insgesamt weniger größere Kristalle gebildet. An diesen haftet weniger Mutterlauge an, insbesondere kann keine Mutterlauge in Agglomeraten feinster Kristalle "eingeschlossen" werden, bzw. die anhaftende Mutterlauge kann leicht abgetrennt werden, z. B. durch einfaches Filtrieren oder Zentrifugieren. Der Reinigungsaufwand verringert sich dadurch erheblich. Auch wird durch diese "Vakuumkühlungskristallisation" eine Verkrustung der Wände des Kristallisators wirksam vermieden.

Das wässrige MGDN enthaltende Gemisch kann durch Verdampfung von Wasser abgekühlt werden, wobei die MGDN-Konzentration des Gemischs im Wesentlichen konstant gehalten wird. Diese Variante kann auch als "Vakuumkühlkristallisation" bezeichnet werden, die mit totalem Rücklauf durchgeführt wird. Das wässrige Gemisch kann durch Verdampfen von Wasser aufkonzentriert werden, wobei die Temperatur des Gemischs im Wesentlichen konstant gehalten wird. Diese Variante kann auch als "isotherme Verdampfungskristallisation" bezeichnet werden. Beide Vorgänge, also sowohl Abkühlung als auch Aufkonzentrierung des wässrigen Gemischs, können aber auch gleichzeitig oder nacheinander erfolgen.

Bei einer bestimmten Temperatur des wässrigen Gemischs, im Allgemeinen unterhalb von etwa 30°C, kann wegen des dann niedrigen Wasserdampfdrucks die Wärme nicht mehr durch Verdampfungskühlung allein, sondern über die Behälterwände des Kristallisators, vorzugsweise durch Solekühlung, abgeführt werden.

Die Ausführung des Kristallisators ist beliebig. Es kann sich dabei z.B. um einen Rührkessel-, Zwangsumlauf-, Leitrohr- oder Fließbett-Kristallisator, beispielsweise vom Oslo-Typ, handeln.

Die Erfindung wird durch die nachfolgenden Beispiele näher erläutert.

### Beispiele

### Vergleichsbeispiel 1

148 g (1,0 mol) reines MGDN werden bei ca. 80°C unter kräftigem Rühren in 608 g (3,04 mol) 20 gew.-%iger Natronlauge innerhalb von ca. 2 h eingetragen. Anschließend wird unter Stickstoff 3 h lang bei 80°C weitergerührt. Danach wird bei 95°C ca. 5 h lang mit Stickstoff gestrippt. Währenddessen wird die Feststoffkonzentration durch Zugabe von Wasser bei unter 45 Gew.-% gehalten. Es resultiert eine dunkelbraune Lösung (Farbzahl nach Hazen > 1000) mit folgender Zusammensetzung: MGDA-Na₃: 230 g (0,85 mol, Ausbeute = 85%), entsprechend 657 g einer ca. 35 gew.-%igen MGDA-Na₃-Lösung; NTA-Na₃: 1,9 Gew.-%; IDA-Na₂: 3,7 Gew.-%; Na₂CO₃: 1,5 Gew.-%; NaOH: 0,2 Gew.-%; Na-Acetat: 0,4 Gew.-%; Na-Formiat: 0,3 Gew.-%; Na-Glykolat: 0,5 Gew.%; Na-Lactat: 0,4 Gew.-%; Na-Glycinat: 0,2 Gew.-%; Na-Alaninat: 0,3 Gew.-%; Acetaldehyd: 1,5 Gew.-%; Wasser: 55 Gew.-%.

### Vergleichsbeispiel 2

148 g (1,0 mol) reines MGDN werden bei ca. 40°C unter kräftigem Rühren in 608 g (3,04 mol) 20 gew.-%iger Natronlauge innerhalb von ca. 2 h eingetragen. Anschließend wird unter Stickstoff 3 h lang bei 40°C weitergerührt. Danach wird bei 95°C ca. 5 h lang mit Stickstoff gestrippt. Währenddessen wird die Feststoffkonzentration durch Zugabe von Wasser unter 45 Gew.-% gehalten. Es resultiert eine braune Lösung (Farbzahl nach Hazen: 445) mit folgender Zusammensetzung: MGDA-Na₃: 247 g (0,91 mol, Ausbeute = 91%), entsprechend 705 g einer ca. 35%igen MGDA-Na₃-Lösung; NTA-Na₃: 0,3 Gew.-%; IDA-Na₂: 2,5 Gew.-%; Na₂CO₃: 0,3 Gew.-%; NaOH: 0,3 Gew.-%; Na-Acetat: 0,2 Gew.-%; Na-Formiat: 0,15 Gew.-%; Na-Glykolat: 0,2 Gew.-%; Na-Lactat: 0,1%; Na-Glycinat: 0,1 Gew.-%; Na-Alaninat: 0,1 Gew.-%; Acetaldehyd: 720 ppm; Wasser: 60 Gew.-%.

### Beispiel 1

148 g (1,0 mol) reines MGDN werden bei ca. 25°C unter kräftigem Rühren in 608 g (3,04 mol) 20 gew.-%iger Natronlauge innerhalb von ca. 2 h eingetragen. Anschließend wird unter Stickstoff zuerst 3 h bei 30°C und dann 2 h bei 40°C weitergerührt. Danach wird bei 95°C innerhalb von ca. 5 h mit Stickstoff gestrippt. Währenddessen wird die Feststoffkonzentration durch Zugabe von Wasser unter 45 Gew.-% gehalten. Es resultiert eine gelb-orange Lösung (Farbzahl nach Hazen: 95) mit folgender Zusammensetzung: MGDA-Na₃: 260 g (0,96 mol, Ausbeute = 96%), entsprechend 650 g einer ca. 40 gew.-%igen MGDA-Na₃-Lösung; NTA-Na₃: < 0,1 Gew.-%; IDA-Na₂: 0,8 Gew.-%; Na₂CO₃: 0,1 Gew.-%; NaOH: 0,2 Gew.-%; Na-Acetat: 0,06 Gew.-%; Na-Formiat: 0,07 Gew.-%; Na-Glykolat: 0,07 Gew.-%; Na-Lactat 0,0 Gew.-%; Na-Glycinat: 0,06 Gew.-%; Na-Alaninat: 0,1 Gew.-%; Acetaldehyd: 80 ppm; Wasser: 58 Gew.-%

### Beispiel 2

148 g (1,0 mol) reines MGDN werden bei ca. 25°C unter kräftigem Rühren in 608 g (3,04 mol) 20 gew.-%iger Natronlauge innerhalb von ca. 2 h eingetragen. Anschließend wird unter Stickstoff zuerst 3 h bei 30°C und dann 2 h bei 40°C weitergerührt. Im Unterschied zu Beispiel 1 wird jetzt 15 Minuten lang unter Druck im Rohrreaktor auf 170°C erhitzt. Danach wird bei 100 - 104°C innerhalb von ca. 5 h mit Stickstoff gestrippt. Währenddessen wird die Feststoffkonzentration durch Zugabe von Wasser unter 45 Gew.-% gehalten. Es resultiert eine gelb-orange Lösung (Farbzahl nach Hazen: 105) mit folgender Zusammensetzung: MGDA-Na₃: 257 g (0,95 mol, Ausbeute = 95%), entsprechend 643 g einer 40 gew.-%igen MGDA-Na₃-Lösung;

Acetaldehyd: < 10 ppm

## Patentansprüche

1. Verfahren zur Herstellung von nebenproduktarmem, farbhellem Methylglycin-N,N-diessigsäure,Trialkalimetallsalz durch alkalische Hydrolyse von Methylglycinnitrildiacetonitril (MGDN) mit den Schritten in der Reihenfolge (a) bis (f):
(a) Mischen von MGDN mit wässriger Alkalilauge bei einer Temperatur ≤ 30°C;
(b) Reagieren lassen der wässrigen alkalischen MGDN-Suspension bei einer Temperatur im Bereich von 10 bis 30°C während eines Zeitraums von 0,1 bis 10 h, wobei eine Lösung gebildet wird;
(c) Reagieren lassen der Lösung aus Schritt (b) bei einer Temperatur im Bereich von 30 bis 40°C während eines Zeitraums von 0,1 bis 10 h;
(d) optional Reagieren lassen der Lösung aus Schritt (c) bei einer Temperatur im Bereich von 50 bis 80°C während eines Zeitraums von 0,5 bis 2 h;
(e) optional Reagieren lassen der Lösung aus Schritt (c) bzw. (d) bei einer Temperatur im Bereich von 110 bis 200°C während eines Zeitraums von 5 bis 60 min;
(f) Hydrolyse und Abtrennung von Ammoniak von der in Schritt (c), (d) bzw. (e) erhaltenen Lösung durch Strippen bei einer Temperatur von 90 bis 105°C.

2. Verfahren nach Anspruch 1, **dadurch gekennzeichnet, dass** in Schritt (b) die Temperatur 25 bis 30°C und in Schritt (c) die Temperatur 35 bis 40°C beträgt.

3. Verfahren nach Anspruch 1 oder 2, **dadurch gekennzeichnet, dass** als wässrige Alkalilauge eine 5 bis 50 gew.-%ige Natronlauge eingesetzt wird.

4. Verfahren nach einem der Ansprüche 1 bis 3, **dadurch gekennzeichnet, dass** das Verfahren diskontinuierlich, halbkontinuierlich oder kontinuierlich durchgeführt wird.

## Claims

1. A process for preparing low-by-product, light-color methylglycine-N,N-diacetic acid tri(alkali metal) salt by alkaline hydrolysis of methylglycinenitrilediacetonitrile (MGDN), comprising the steps in the sequence (a) to (f):
(a) mixing of MGDN with aqueous alkali at a temperature of ≤30°C;
(b) allowing the aqueous alkaline MGDN suspension to react at a temperature in the range from 10 to 30°C over a period of from 0.1 to 10 h to form a solution;
(c) allowing the solution from step (b) to react at a temperature in the range from 30 to 40°C over a period of from 0.1 to 10 h;
(d) optionally allowing the solution from step (c) to react at a temperature in the range from 50 to 80°C over a period of from 0.5 to 2 h;
(e) optionally allowing the solution from step (c) or (d) to react at a temperature in the range from 110 to 200°C over a period of from 5 to 60 min;
(f) hydrolysis and removal of ammonia of the solution obtained in step (c), (d) or (e) by stripping at a temperature of from 90 to 105°C.

2. The process according to claim 1, wherein the temperature in step (b) is from 25 to 30°C and the temperature in step (c) is from 35 to 40°C.

3. The process according to claim 1 or 2, wherein the aqueous alkali used is from 5 to 50% by weight sodium hydroxide solution.

4. The process according to any of claims 1 to 3, which is carried out batchwise, semicontinuously or continuously.

## Revendications

1. Procédé de fabrication d'un sel métallique trialcalin de l'acide méthylglycine-N,N-diacétique pauvre en sous-produits et de couleur claire par hydrolyse alcaline de méthylglycinenitrilediacétonitrile (MGDN) comprenant les étapes successives (a) à (f) :
(a) le mélange de MGDN avec une lessive alcaline aqueuse à une température ≤ 30 °C ;
(b) la mise en réaction de la suspension de MGDN alcaline aqueuse à une température dans la plage allant de 10 à 30°C pendant une durée de 0,1 à 10 h, une solution étant formée ;
(c) la mise en réaction de la solution de l'étape (b) à une température dans la plage allant de 30 à 40°C pendant une durée de 0,1 à 10 h ;
(d) éventuellement la mise en réaction de la solution de l'étape (c) à une température dans la plage allant de 50 à 80°C pendant une durée de 0,5 à 2 h ;
(e) éventuellement la mise en réaction de la solution de l'étape (c) ou (d) à une température dans la plage allant de 110 à 200°C pendant une durée de 5 à 60 min ;
(f) l'hydrolyse et la séparation de l'ammoniac de la solution obtenue à l'étape (c), (d) ou (e) par extraction à une température de 90 à 105°C.

2. Procédé selon la revendication 1, **caractérisé en ce que** la température est de 25 à 30°C à l'étape (b) et la température est de 35 à 40°C à l'étape (c).

3. Procédé selon la revendication 1 ou 2, **caractérisé en ce qu'**une lessive de soude de 5 à 50 % en poids est utilisée en tant que lessive alcaline aqueuse.

4. Procédé selon l'une quelconque des revendications 1 à 3, **caractérisé en ce que** le procédé est réalisé de manière discontinue, semi-continue ou continue.
